(19) Europäisches Patentamt
European Patent Office
Office européen des brevets

(11) Veröffentlichungsnummer: **0 410 265 B1**

# (12) EUROPÄISCHE PATENTSCHRIFT

(45) Veröffentlichungstag der Patentschrift: **14.12.94**

(51) Int. Cl.[5]: **C07D 513/04**, A01N 43/90, //(C07D513/04,285:00,237:00)

(21) Anmeldenummer: **90113631.7**

(22) Anmeldetag: **17.07.90**

Die Akte enthält technische Angaben, die nach dem Eingang der Anmeldung eingereicht wurden und die nicht in dieser Patentschrift enthalten sind.

## (54) Zimtsäureester.

(30) Priorität: **26.07.89 DE 3924719**

(43) Veröffentlichungstag der Anmeldung: **30.01.91 Patentblatt 91/05**

(45) Bekanntmachung des Hinweises auf die Patenterteilung: **14.12.94 Patentblatt 94/50**

(84) Benannte Vertragsstaaten: **AT BE CH DE ES FR GB IT LI NL**

(56) Entgegenhaltungen:
**EP-A- 0 053 321**
**EP-A- 0 238 711**
**DE-A- 3 724 399**

(73) Patentinhaber: **BASF Aktiengesellschaft**
**Carl-Bosch-Strasse 38**
**D-67063 Ludwigshafen (DE)**

(72) Erfinder: **Rueb, Lothar, Dr.**
**Am Schoeneck 11**
**D-6720 Speyer (DE)**
Erfinder: **Eicken, Karl, Dr.**
**Am Huettenwingert 12**
**D-6706 Wachenheim (DE)**
Erfinder: **Westphalen, Karl-Otto, Dr.**
**Masubergweg 58**
**D-6720 Speyer (DE)**
Erfinder: **Wuerzer, Bruno, Dr.**
**Ruedigerstrasse 13**
**D-6701 Otterstadt (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 410 265 B1

Rank Xerox (UK) Business Services
(3.10/3.09/3.3.3)

## Beschreibung

Die vorliegende Erfindung betrifft neue Zimtsäureester der allgemeinen Formel I,

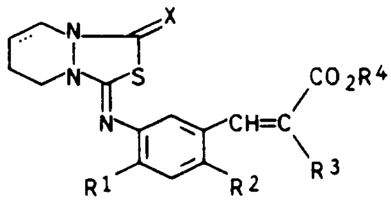

I

in der die punktierte Bindung für eine Einfach- oder Doppelbindung steht und die Substituenten folgende Bedeutung haben:

$R^1$
: Wasserstoff oder Fluor,

$R^2$
: Halogen,

$R^3$
: Wasserstoff, Halogen oder eine $C_1$-$C_4$-Alkylgruppe,

$R^4$
: Wasserstoff, eine $C_1$-$C_6$-Alkylgruppe, welche durch ein oder zwei $C_1$-$C_4$-Alkoxygruppen substituiert sein kann, eine $C_3$-$C_6$-Alkenylgruppe, eine $C_3$-$C_6$-Alkinylgruppe oder ein Benzylrest, und

X
: Sauerstoff oder Schwefel.

Des weiteren betrifft die Erfindung Verfahren zur Herstellung dieser Verbindungen sowie deren Verwendung als herbizide Mittel.

In der EP-A-0 238 711 werden herbizide Thiadiazabicyclononan derivate beschrieben.

Aus der JP-A 27962/1986 sind 5-(N-Tetrahydrophthalimido)-zimtsäurederivate als Herbizide bekannt, welche jedoch hinsichtlich der benötigten Aufwandmengen zu wünschen übrig lassen. Der Erfindung lag daher die Aufgabe zugrunde, besonders aktive herbizide Verbindungen bereitzustellen.

Demgemäß wurden die eingangs definierten Zimtsäureester I gefunden.

Man erhält die Verbindungen I beispielsweise dadurch, daß man ein Aminozimtsäurederivat der allgemeinen Formel II in an sich bekannter Weise (Houben-Weyl, Bd. IX, S. 867 f. (1955)) in einem inerten organischen Lösungsmittel mit Thiophosgen in das entsprechende Isothiocyanat III überführt, III anschließend in einem aprotisch polaren Lösungsmittel an ein Tetrahydro- oder Perhydrodiazinderivat IV addiert und den so erhaltenen Thioharnstoff V mit einem Phosgenierungsmittel oder Thiophosgenierungsmittel (Phos.) zu I cyclisiert.

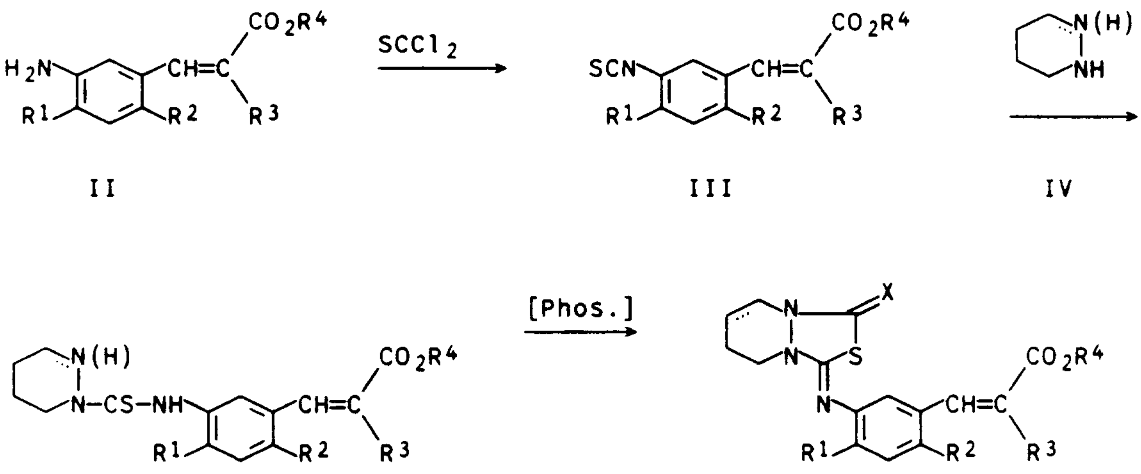


II III IV

V I

Die Umsetzung des Aminozimtsäurederivats II mit Thiophosgen erfolgt in der Regel bei Temperaturen von -50 ° C bis 100 ° C, vorzugsweise 0 ° C bis 50 ° C.

Diese Umsetzung kann sowohl in einem Zweiphasen-Lösungsmittelsystem wie Methylenchlorid/Wasser als auch in Gegenwart einer Base in einem aprotisch polaren organischen Lösungsmittel durchgeführt werden.

Im letztgenannten Fall arbeitet man bevorzugt in Toluol in Gegenwart einer organischen Base, vorzugsweise eines tertiären Amins wie Triethylamin.

Die für die Umsetzung benötigten Aminozimtsäurederivate erhält man beispielsweise durch Reduktion aus den entsprechenden Nitrozimtsäurederivaten nach bekannten Methoden (DE-A 37 24 399, DE-A 36 03 789).

Die Umsetzung der Isothiocyanate III mit den Piperazinen IV erfolgt bevorzugt in aprotisch polaren Lösungsmitteln wie vorzugsweise Ethern, insbesondere Tetrahydrofuran bei Temperaturen von -50 ° C bis 100 ° C, vorzugsweise 0 ° C bis 50 ° C.

Die anschließende Cyclisierung des Thioharnstoffs V mit einem Phosgenierungsmittel oder Thiophosgenierungsmittel geschieht in der Regel bei Temperaturen von 0 ° C bis 100 ° C, vorzugsweise 20 ° C bis 70 ° C in einem aprotisch polaren Lösungsmittel in Gegenwart einer Base.

Als Phosgenierungsmittel und Thiophosgenierungsmittel eignen sich insbesondere Phosgen, Thiophosgen und Chlorameisensäuretrichlormethylester.
Als Lösungsmittel dienen vorzugsweise Ether, Halogenkohlenwasserstoffe und Kohlenwasserstoffe, insbesondere Halogenkohlenwasserstoffe wie Methylenchlorid.

Geeignete Basen sind teritäre Amine wie insbesondere Pyridin.

Man erhält die Verbindungen der Formel I aber auch, wenn man ein Anilinderivat der Formel VI entsprechend den vorstehend geschilderten Bedingungen zuerst mit Thiophosgen in das entsprechende Isothiocyanat IIIa überführt, IIIa an ein Piperazin IV addiert, das so erhaltene Thioharnstoffderivat Va unter saurer Spaltung der Acetalgruppe zur Aldehydfunktion mit einem Phosgenierungsmittel zum Aldehyd VII cyclisiert und VII mit einem Phosphor-Ylid der Formel VIII umsetzt.

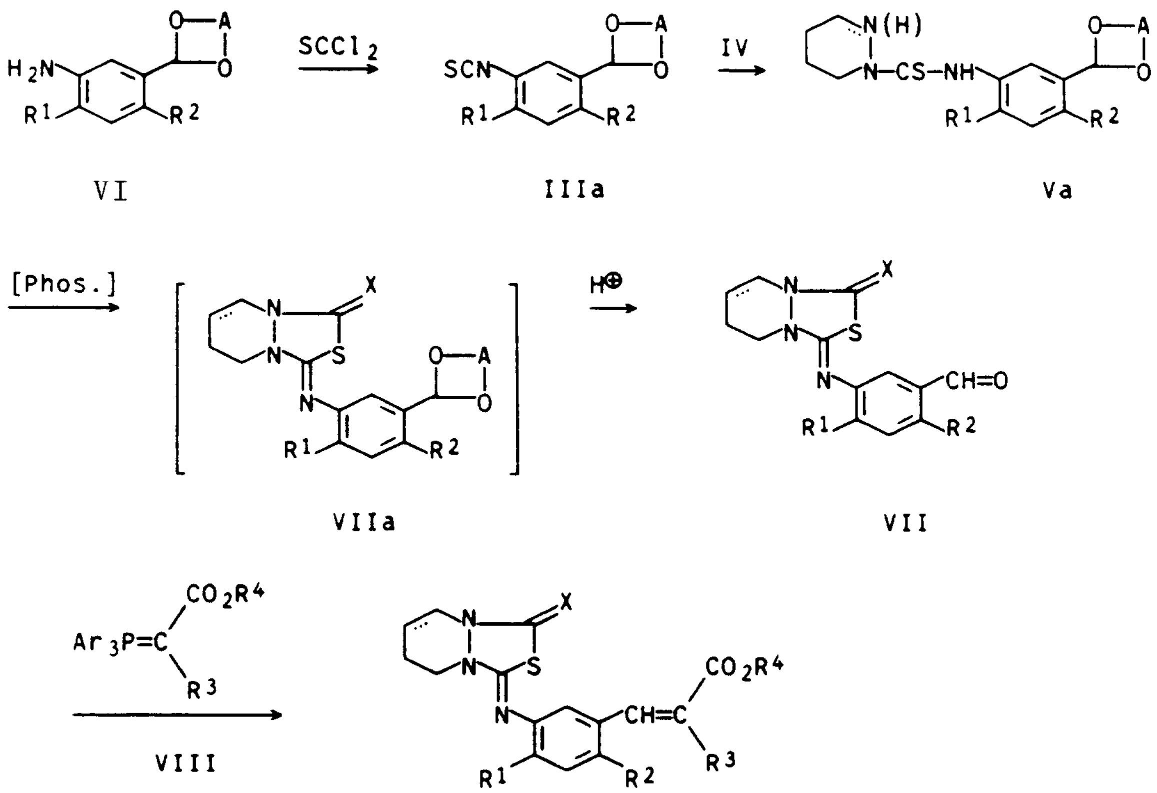


VI IIIa Va

VIIa VII

I

In den Formeln VI, IIa, Va und VIIa bedeutet A eine Ethylen- oder Propyleneinheit, welche ein bis drei Alkylgruppen wie Methyl, Ethyl, Propyl und 1-Methylethyl, vorzugsweise Methyl tragen kann.

Die Umsetzungen von VI zu IIIa, von IIIa zu Va und von Va zu VIIa verlaufen unter den vorstehend geschilderten Bedingungen analog zur ersten Synthesevariante.

Die Acetalgruppe in Verbindung VIIa wird unter sauren Bedingungen beispielsweise in Gegenwart von Mineralsäuren wie Salzsäure und Schwefelsäure oder organischen Säuren wie p-Toluolsulfonsäure in die Aldehydfunktion überführt.

Die Umsetzung des so erhaltenen Aldehyds VII mit dem Phosphor-Ylid VIII erfolgt in an sich bekannter Weise (z.B. DE-A 39 04 082) in einem inerten organischen Lösungsmittel wie Toluol, Tetrahydrofuran, Dimethylformamid, Dimethylsulfoxid und Methanol.

Ar in Formel VIII bedeutet einen unsubstituierten oder substituierten Arylrest, wobei im allgemeinen der Phenylrest bevorzugt ist.

Man erhält die für die Umsetzung benötigten Phosphor-Ylide VIII analog literaturbekannten Methoden (z.B. Chem. Ber. 95, 3003 (1962)).

Im Hinblick auf die bestimmungsgemäße Verwendung der Verbindungen I als Herbizide kommen als Substituenten vorzugsweise folgende Reste in Betracht:

$R^1$

Wasserstoff und Fluor;

$R^2$

Halogen wie Fluor, Chlor und Brom, insbesondere Chlor;

$R^3$

Wasserstoff; ein Halogenatom wie unter $R^2$ genannt, insbesondere Chlor und Brom; eine Alkylgruppe wie Methyl, Ethyl, Propyl, 1-Methylethyl, Butyl, 1-Methylpropyl, 2-Methylpropyl und 1,1-Dimethylethyl, insbesondere Methyl und Ethyl;

$R^4$

Wasserstoff; eine Alkylgruppe wie bei $R^3$ genannt sowie n-Pentyl, 1-Methylbutyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 1-Methylpentyl, 2-Methylpentyl, 3-Methylpentyl, 4-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 2-Ethylbutyl und 1-Ethyl-2-methylpropyl, insbesondere

Methyl, Ethyl, Propyl und iso-Propyl, wobei diese Alkylgruppe durch ein bis zwei $C_1$-$C_4$-Alkoxygruppen substituiert sein kann, wobei Alkoxyalkylgruppen wie Methoxymethyl, 2-Methoxyethyl, 2-Methoxypropyl, 3-Methoxypropyl, 2-Methoxy-1-methylethyl, Ethoxymethyl, 2-Ethoxyethyl, 2-Ethoxypropyl, 3-Ethoxypropyl, 2-Ethoxyl-methylethyl und 1-Ethoxy-1-methylethyl, insbesondere Methoxyethyl und Ethoxyethyl;
eine Alkenylgruppe wie 2-Propenyl, 2-Butenyl, 3-Butenyl, 1-Methyl-2-propenyl, 2-Methyl-2-propenyl, 2-Pentenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-2-propenyl, 1-Ethyl-2-propenyl, 2-Hexenyl, 3-Hexenyl, 4-Hexenyl, 5-Hexenyl, 1-Methyl-2-pentenyl, 2-Methyl-2-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-2-butenyl, 1,2-Dimethyl-3-butenyl, 1,3-Dimethyl-2-butenyl, 1,2-Dimethyl-2-butenyl, 2,2-Dimethyl-3-butenyl, 2,3-Dimethyl-2-butenyl, 2,3-Dimethyl-3-butenyl, 1-Ethyl-2-butenyl, 1-Ethyl-3-butenyl, 2-Ethyl-2-butenyl, 2-Ethyl-3-butenyl, 1,1,2-Trimethyl-2-propenyl, 1-Ethyl-1-methyl-2-propenyl und 1-Ethyl-2-methyl-2-propenyl, oder eine entsprechende Alkenyloxygruppe,
eine Alkinylgruppe wie 2-Propinyl, 2-Butinyl, 3-Butinyl, 1-Methyl-2-propinyl, 2-Pentinyl, 3-Pentinyl, 4-Pentinyl, 1-Methyl-3-butinyl, 2-Methyl-3-butinyl, 1-Methyl-2-butinyl, 1,1-Dimethyl-2-propinyl, 1-Ethyl-2-propinyl, 2-Hexinyl, 3-Hexinyl, 4-Alkinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-4-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 1-Ethyl-2-butinyl, 1-Ethyl-3-butinyl, 2-Ethyl-3-butinyl und 1-Ethyl-1-methyl-2-propinyl, oder eine entsprechende Alkinyloxygruppe, oder ein Benzylrest.

Beispiele für sehr aktive Verbindungen I sind in den nachstehenden Tabellen A und B aufgeführt.

**Tabelle A:**

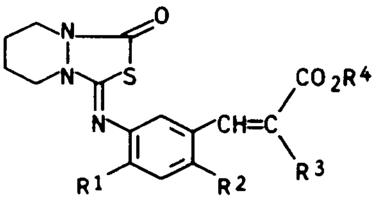

oder

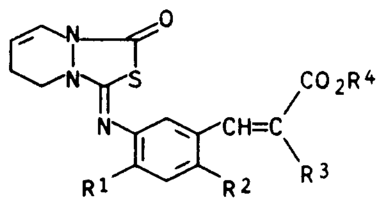

| $R^1$ | $R^2$ | $R^3$ | $R^4$ |
|---|---|---|---|
| H | Cl | H | H |
| F | Cl | H | H |
| H | Br | H | H |
| F | Br | H | H |
| H | Cl | Cl | H |
| F | Cl | Cl | H |
| H | Br | Cl | H |
| F | Br | Cl | H |
| H | Cl | Br | H |
| F | Cl | Br | H |
| H | Br | Br | H |
| F | Br | Br | H |
| H | Cl | $CH_3$ | H |
| F | Cl | $CH_3$ | H |
| H | Br | $CH_3$ | H |
| F | Br | $CH_3$ | H |
| H | Cl | $CH_2CH_3$ | H |
| F | Cl | $CH_2CH_3$ | H |
| H | Br | $CH_2CH_3$ | H |
| F | Br | $CH_2CH_3$ | H |
| H | Cl | H | $CH_3$ |
| F | Cl | H | $CH_3$ |
| H | Br | H | $CH_3$ |
| F | Br | H | $CH_3$ |
| H | Cl | Cl | $CH_3$ |
| F | Cl | Cl | $CH_3$ |
| H | Br | Cl | $CH_3$ |
| F | Br | Cl | $CH_3$ |
| H | Cl | Br | $CH_3$ |
| F | Cl | Br | $CH_3$ |
| H | Br | Br | $CH_3$ |
| F | Br | Br | $CH_3$ |

Tabelle A (Fortsetzung)

| $R^1$ | $R^2$ | $R^3$ | $R^4$ |
|---|---|---|---|
| H | Cl | $CH_3$ | $CH_3$ |
| F | Cl | $CH_3$ | $CH_3$ |
| H | Br | $CH_3$ | $CH_3$ |
| F | Br | $CH_3$ | $CH_3$ |
| H | Cl | $CH_2CH_3$ | $CH_3$ |
| F | Cl | $CH_2CH_3$ | $CH_3$ |
| H | Br | $CH_2CH_3$ | $CH_3$ |
| F | Br | $CH_2CH_3$ | $CH_3$ |
| H | Cl | H | $CH_2CH_3$ |
| F | Cl | H | $CH_2CH_3$ |
| H | Br | H | $CH_2CH_3$ |
| F | Br | H | $CH_2CH_3$ |
| H | Cl | Cl | $CH_2CH_3$ |
| F | Cl | Cl | $CH_2CH_3$ |
| H | Br | Cl | $CH_2CH_3$ |
| F | Br | Cl | $CH_2CH_3$ |
| H | Cl | Br | $CH_2CH_3$ |
| F | Cl | Br | $CH_2CH_3$ |
| H | Br | Br | $CH_2CH_3$ |
| F | Br | Br | $CH_2CH_3$ |
| H | Cl | $CH_3$ | $CH_2CH_3$ |
| F | Cl | $CH_3$ | $CH_2CH_3$ |
| H | Br | $CH_3$ | $CH_2CH_3$ |
| F | Br | $CH_3$ | $CH_2CH_3$ |
| H | Cl | $CH_2CH_3$ | $CH_2CH_3$ |
| F | Cl | $CH_2CH_3$ | $CH_2CH_3$ |
| H | Br | $CH_2CH_3$ | $CH_2CH_3$ |
| F | Br | $CH_2CH_3$ | $CH_2CH_3$ |
| H | Cl | H | $(CH_2)_2CH_3$ |
| F | Cl | H | $(CH_2)_2CH_3$ |
| H | Cl | Cl | $(CH_2)_2CH_3$ |
| F | Cl | Cl | $(CH_2)_2CH_3$ |
| H | Cl | Br | $(CH_2)_2CH_3$ |
| F | Cl | Br | $(CH_2)_2CH_3$ |
| H | Cl | $CH_3$ | $(CH_2)_2CH_3$ |
| F | Cl | $CH_3$ | $(CH_2)_2CH_3$ |
| H | Cl | $CH_2CH_3$ | $(CH_2)_2CH_3$ |
| F | Cl | $CH_2CH_3$ | $(CH_2)_2CH_3$ |
| H | Cl | H | $CH(CH_3)_2$ |
| F | Cl | H | $CH(CH_3)_2$ |
| H | Cl | Cl | $CH(CH_3)_2$ |

Tabelle A (Fortsetzung)

| $R^1$ | $R^2$ | $R^3$ | $R^4$ |
|---|---|---|---|
| F | Cl | Cl | $CH(CH_3)_2$ |
| H | Cl | Br | $CH(CH_3)_2$ |
| F | Cl | Br | $CH(CH_3)_2$ |
| H | Cl | $CH_3$ | $CH(CH_3)_2$ |
| F | Cl | $CH_3$ | $CH(CH_3)_2$ |
| H | Cl | $CH_2CH_3$ | $CH(CH_3)_2$ |
| F | Cl | $CH_2CH_3$ | $CH(CH_3)_2$ |
| H | Cl | H | $(CH_2)_3CH_3$ |
| F | Cl | H | $(CH_2)_3CH_3$ |
| H | Cl | Cl | $(CH_2)_3CH_3$ |
| F | Cl | Cl | $(CH_2)_3CH_3$ |
| H | Cl | Br | $(CH_2)_3CH_3$ |
| F | Cl | Br | $(CH_2)_3CH_3$ |
| H | Cl | $CH_3$ | $(CH_2)_3CH_3$ |
| F | Cl | $CH_3$ | $(CH_2)_3CH_3$ |
| H | Cl | $CH_2CH_3$ | $(CH_2)_3CH_3$ |
| F | Cl | $CH_2CH_3$ | $(CH_2)_3CH_3$ |
| H | Cl | H | $CH_2CH(CH_3)_2$ |
| F | Cl | H | $CH_2CH(CH_3)_2$ |
| H | Cl | Cl | $CH_2CH(CH_3)_2$ |
| F | Cl | Cl | $CH_2CH(CH_3)_2$ |
| H | Cl | Br | $CH_2CH(CH_3)_2$ |
| F | Cl | Br | $CH_2CH(CH_3)_2$ |
| H | Cl | $CH_3$ | $CH_2CH(CH_3)_2$ |
| F | Cl | $CH_3$ | $CH_2CH(CH_3)_2$ |
| H | Cl | $CH_2CH_3$ | $CH_2CH(CH_3)_2$ |
| F | Cl | $CH_2CH_3$ | $CH_2CH(CH_3)_2$ |
| H | Cl | H | $(CH_2)_4CH_3$ |
| F | Cl | H | $(CH_2)_4CH_3$ |
| H | Cl | Cl | $(CH_2)_4CH_3$ |
| F | Cl | Cl | $(CH_2)_4CH_3$ |
| H | Cl | Br | $(CH_2)_4CH_3$ |
| F | Cl | Br | $(CH_2)_4CH_3$ |
| H | Cl | $CH_3$ | $(CH_2)_4CH_3$ |
| F | Cl | $CH_3$ | $(CH_2)_4CH_3$ |
| H | Cl | $CH_2CH_3$ | $(CH_2)_4CH_3$ |
| F | Cl | $CH_2CH_3$ | $(CH_2)_4CH_3$ |
| H | Cl | H | $(CH_2)_2CH(CH_3)_2$ |
| F | Cl | H | $(CH_2)_2CH(CH_3)_2$ |
| H | Cl | Cl | $(CH_2)_2CH(CH_3)_2$ |
| F | Cl | Cl | $(CH_2)_2CH(CH_3)_2$ |

Tabelle A (Fortsetzung)

| $R^1$ | $R^2$ | $R^3$ | $R^4$ |
|---|---|---|---|
| H | Cl | Br | $(CH_2)_2CH(CH_3)_2$ |
| F | Cl | Br | $(CH_2)_2CH(CH_3)_2$ |
| H | Cl | $CH_3$ | $(CH_2)_2CH(CH_3)_2$ |
| F | Cl | $CH_3$ | $(CH_2)_2CH(CH_3)_2$ |
| H | Cl | $CH_2CH_3$ | $(CH_2)_2CH(CH_3)_2$ |
| F | Cl | $CH_2CH_3$ | $(CH_2)_2CH(CH_3)_2$ |
| H | Cl | H | $(CH_2)_2OCH_3$ |
| F | Cl | H | $(CH_2)_2OCH_3$ |
| H | Cl | Cl | $(CH_2)_2OCH_3$ |
| F | Cl | Cl | $(CH_2)_2OCH_3$ |
| H | Cl | Br | $(CH_2)_2OCH_3$ |
| F | Cl | Br | $(CH_2)_2OCH_3$ |
| H | Cl | $CH_3$ | $(CH_2)_2OCH_3$ |
| F | Cl | $CH_3$ | $(CH_2)_2OCH_3$ |
| H | Cl | $CH_2CH_3$ | $(CH_2)_2OCH_3$ |
| F | Cl | $CH_2CH_3$ | $(CH_2)_2OCH_3$ |
| H | Cl | H | $CH(CH_3)CH_2OCH_3$ |
| F | Cl | H | $CH(CH_3)CH_2OCH_3$ |
| H | Cl | Cl | $CH(CH_3)CH_2OCH_3$ |
| F | Cl | Cl | $CH(CH_3)CH_2OCH_3$ |
| H | Cl | Br | $CH(CH_3)CH_2OCH_3$ |
| F | Cl | Br | $CH(CH_3)CH_2OCH_3$ |
| H | Cl | $CH_3$ | $CH(CH_3)CH_2OCH_3$ |
| F | Cl | $CH_3$ | $CH(CH_3)CH_2OCH_3$ |
| H | Cl | $CH_2CH_3$ | $CH(CH_3)CH_2OCH_3$ |
| F | Cl | $CH_2CH_3$ | $CH(CH_3)CH_2OCH_3$ |
| H | Cl | H | $CH_2CH=CH_2$ |
| F | Cl | H | $CH_2CH=CH_2$ |
| H | Cl | Cl | $CH_2CH=CH_2$ |
| F | Cl | Cl | $CH_2CH=CH_2$ |
| H | Cl | Br | $CH_2CH=CH_2$ |
| F | Cl | Br | $CH_2CH=CH_2$ |
| H | Cl | $CH_3$ | $CH_2CH=CH_2$ |
| F | Cl | $CH_3$ | $CH_2CH=CH_2$ |
| H | Cl | $CH_2CH_3$ | $CH_2CH=CH_2$ |
| F | Cl | $CH_2CH_3$ | $CH_2CH=CH_2$ |
| H | Cl | H | $CH_2CH=CHCH_3$ |
| F | Cl | H | $CH_2CH=CHCH_3$ |
| H | Cl | Cl | $CH_2CH=CHCH_3$ |
| F | Cl | Cl | $CH_2CH=CHCH_3$ |
| H | Cl | Br | $CH_2CH=CHCH_3$ |
| F | Cl | Br | $CH_2CH=CHCH_3$ |

Tabelle A (Fortsetzung)

| $R^1$ | $R^2$ | $R^3$ | $R^4$ |
|---|---|---|---|
| H | Cl | $CH_3$ | $CH_2CH=CHCH_3$ |
| F | Cl | $CH_3$ | $CH_2CH=CHCH_3$ |
| H | Cl | $CH_2CH_3$ | $CH_2CH=CHCH_3$ |
| F | Cl | $CH_2CH_3$ | $CH_2CH=CHCH_3$ |
| H | Cl | H | $CH_2C{\equiv}CH$ |
| F | Cl | H | $CH_2C{\equiv}CH$ |
| H | Cl | Cl | $CH_2C{\equiv}CH$ |
| F | Cl | Cl | $CH_2C{\equiv}CH$ |
| H | Cl | Br | $CH_2C{\equiv}CH$ |
| F | Cl | Br | $CH_2C{\equiv}CH$ |
| H | Cl | $CH_3$ | $CH_2C{\equiv}CH$ |
| F | Cl | $CH_3$ | $CH_2C{\equiv}CH$ |
| H | Cl | $CH_2CH_3$ | $CH_2C{\equiv}CH$ |
| F | Cl | $CH_2CH_3$ | $CH_2C{\equiv}CH$ |
| H | Cl | H | $CH_2C{\equiv}CCH_3$ |
| F | Cl | H | $CH_2C{\equiv}CCH_3$ |
| H | Cl | Cl | $CH_2C{\equiv}CCH_3$ |
| F | Cl | Cl | $CH_2C{\equiv}CCH_3$ |
| H | Cl | Br | $CH_2C{\equiv}CCH_3$ |
| F | Cl | Br | $CH_2C{\equiv}CCH_3$ |
| H | Cl | $CH_3$ | $CH_2C{\equiv}CCH_3$ |
| F | Cl | $CH_3$ | $CH_2C{\equiv}CCH_3$ |
| H | Cl | $CH_2CH_3$ | $CH_2C{\equiv}CCH_3$ |
| F | Cl | $CH_2CH_3$ | $CH_2C{\equiv}CCH_3$ |
| H | Cl | H | $CH_2Ph$ |
| F | Cl | H | $CH_2Ph$ |
| H | Cl | Cl | $CH_2Ph$ |
| F | Cl | Cl | $CH_2Ph$ |
| H | Cl | Br | $CH_2Ph$ |
| F | Cl | Br | $CH_2Ph$ |
| H | Cl | $CH_3$ | $CH_2Ph$ |
| F | Cl | $CH_3$ | $CH_2Ph$ |
| H | Cl | $CH_2CH_3$ | $CH_2Ph$ |
| F | Cl | $CH_2CH_3$ | $CH_2Ph$ |
| H | F | Cl | $CH_3$ |
| H | F | Br | $CH_3$ |
| H | F | $CH_3$ | $CH_3$ |
| H | F | Cl | $CH_2CH_3$ |
| H | F | Br | $CH_2CH_3$ |
| H | F | $CH_3$ | $CH_2CH_3$ |
| H | F | Cl | $(CH_2)_2CH_3$ |

Tabelle A (Fortsetzung)

| $R^1$ | $R^2$ | $R^3$ | $R^4$ |
|---|---|---|---|
| H | F | Br | $(CH_2)_2CH_3$ |
| H | F | $CH_3$ | $(CH_2)_2CH_3$ |
| H | F | Cl | $CH(CH_3)_2$ |
| H | F | Br | $CH(CH_3)_2$ |
| H | F | $CH_3$ | $CH(CH_3)_2$ |
| H | F | Cl | $(CH_2)_3CH_3$ |
| H | F | Br | $(CH_2)_3CH_3$ |
| H | F | $CH_3$ | $(CH_2)_3CH_3$ |
| H | F | Cl | $CH_2CH(CH_3)_2$ |
| H | F | Br | $CH_2CH(CH_3)_2$ |
| H | F | $CH_3$ | $CH_2CH(CH_3)_2$ |
| H | F | Cl | $(CH_2)_4CH_3$ |
| H | F | Br | $(CH_2)_4CH_3$ |
| H | F | $CH_3$ | $(CH_2)_4CH_3$ |
| H | F | Cl | $(CH_2)_2CH(CH_3)_2$ |
| H | F | Br | $(CH_2)_2CH(CH_3)_2$ |
| H | F | $CH_3$ | $(CH_2)_2CH(CH_3)_2$ |
| H | F | Cl | $(CH_2)_2OCH_3$ |
| H | F | Br | $(CH_2)_2OCH_3$ |
| H | F | $CH_3$ | $(CH_2)_2OCH_3$ |
| H | F | Cl | $CH(CH_3)CH_2OCH_3$ |
| H | F | Br | $CH(CH_3)CH_2OCH_3$ |
| H | F | $CH_3$ | $CH(CH_3)CH_2OCH_3$ |
| H | F | Cl | $CH_2CH{=}CH_2$ |
| H | F | Br | $CH_2CH{=}CH_2$ |
| H | F | $CH_3$ | $CH_2CH{=}CH_2$ |
| H | F | Cl | $CH_2CH{=}CHCH_3$ |
| H | F | Br | $CH_2CH{=}CHCH_3$ |
| H | F | $CH_3$ | $CH_2CH{=}CHCH_3$ |
| H | F | Cl | $CH_2C{\equiv}CH$ |
| H | F | Br | $CH_2C{\equiv}CH$ |
| H | F | $CH_3$ | $CH_2C{\equiv}CH$ |
| H | F | Cl | $CH_2C{\equiv}CCH_3$ |
| H | F | Br | $CH_2C{\equiv}CCH_3$ |
| H | F | $CH_3$ | $CH_2C{\equiv}CCH_3$ |
| H | F | Cl | $CH_2Ph$ |
| H | F | Br | $CH_2Ph$ |
| H | F | $CH_3$ | $CH_2Ph$ |

Tabelle B:

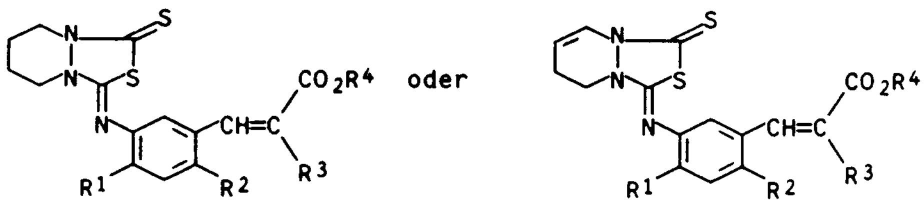

| $R^1$ | $R^2$ | $R^3$ | $R^4$ |
|---|---|---|---|
| H | Cl | H | H |
| F | Cl | H | H |
| H | Br | H | H |
| F | Br | H | H |
| H | Cl | Cl | H |
| F | Cl | Cl | H |
| H | Br | Cl | H |
| F | Br | Cl | H |
| H | Cl | Br | H |
| F | Cl | Br | H |
| H | Br | Br | H |
| F | Br | Br | H |
| H | Cl | $CH_3$ | H |
| F | Cl | $CH_3$ | H |
| H | Br | $CH_3$ | H |
| F | Br | $CH_3$ | H |
| H | Cl | $CH_2CH_3$ | H |
| F | Cl | $CH_2CH_3$ | H |
| H | Br | $CH_2CH_3$ | H |
| F | Br | $CH_2CH_3$ | H |
| H | Cl | H | $CH_3$ |
| F | Cl | H | $CH_3$ |
| H | Br | H | $CH_3$ |
| F | Br | H | $CH_3$ |
| H | Cl | Cl | $CH_3$ |
| F | Cl | Cl | $CH_3$ |
| H | Br | Cl | $CH_3$ |
| F | Br | Cl | $CH_3$ |
| H | Cl | Br | $CH_3$ |
| F | Cl | Br | $CH_3$ |
| H | Br | Br | $CH_3$ |
| F | Br | Br | $CH_3$ |
| H | Cl | $CH_3$ | $CH_3$ |

Tabelle B (Fortsetzung)

| $R^1$ | $R^2$ | $R^3$ | $R^4$ |
|---|---|---|---|
| F | Cl | $CH_3$ | $CH_3$ |
| H | Br | $CH_3$ | $CH_3$ |
| F | Br | $CH_3$ | $CH_3$ |
| H | Cl | $CH_2CH_3$ | $CH_3$ |
| F | Cl | $CH_2CH_3$ | $CH_3$ |
| H | Br | $CH_2CH_3$ | $CH_3$ |
| F | Br | $CH_2CH_3$ | $CH_3$ |
| H | Cl | H | $CH_2CH_3$ |
| F | Cl | H | $CH_2CH_3$ |
| H | Br | H | $CH_2CH_3$ |
| F | Br | H | $CH_2CH_3$ |
| H | Cl | Cl | $CH_2CH_3$ |
| F | Cl | Cl | $CH_2CH_3$ |
| H | Br | Cl | $CH_2CH_3$ |
| F | Br | Cl | $CH_2CH_3$ |
| H | Cl | Br | $CH_2CH_3$ |
| F | Cl | Br | $CH_2CH_3$ |
| H | Br | Br | $CH_2CH_3$ |
| F | Br | Br | $CH_2CH_3$ |
| H | Cl | $CH_3$ | $CH_2CH_3$ |
| F | Cl | $CH_3$ | $CH_2CH_3$ |
| H | Br | $CH_3$ | $CH_2CH_3$ |
| F | Br | $CH_3$ | $CH_2CH_3$ |
| H | Cl | $CH_2CH_3$ | $CH_2CH_3$ |
| F | Cl | $CH_2CH_3$ | $CH_2CH_3$ |
| H | Br | $CH_2CH_3$ | $CH_2CH_3$ |
| F | Br | $CH_2CH_3$ | $CH_2CH_3$ |
| H | Cl | H | $(CH_2)_2CH_3$ |
| F | Cl | H | $(CH_2)_2CH_3$ |
| H | Cl | Cl | $(CH_2)_2CH_3$ |
| F | Cl | Cl | $(CH_2)_2CH_3$ |
| H | Cl | Br | $(CH_2)_2CH_3$ |
| F | Cl | Br | $(CH_2)_2CH_3$ |
| H | Cl | $CH_3$ | $(CH_2)_2CH_3$ |
| F | Cl | $CH_3$ | $(CH_2)_2CH_3$ |
| H | Cl | $CH_2CH_3$ | $(CH_2)_2CH_3$ |
| F | Cl | $CH_2CH_3$ | $(CH_2)_2CH_3$ |
| H | Cl | H | $CH(CH_3)_2$ |
| F | Cl | H | $CH(CH_3)_2$ |
| H | Cl | Cl | $CH(CH_3)_2$ |
| F | Cl | Cl | $CH(CH_3)_2$ |

Tabelle B (Fortsetzung)

| $R^1$ | $R^2$ | $R^3$ | $R^4$ |
|---|---|---|---|
| H | Cl | Br | $CH(CH_3)_2$ |
| F | Cl | Br | $CH(CH_3)_2$ |
| H | Cl | $CH_3$ | $CH(CH_3)_2$ |
| F | Cl | $CH_3$ | $CH(CH_3)_2$ |
| H | Cl | $CH_2CH_3$ | $CH(CH_3)_2$ |
| F | Cl | $CH_2CH_3$ | $CH(CH_3)_2$ |
| H | Cl | H | $(CH_2)_3CH_3$ |
| F | Cl | H | $(CH_2)_3CH_3$ |
| H | Cl | Cl | $(CH_2)_3CH_3$ |
| F | Cl | Cl | $(CH_2)_3CH_3$ |
| H | Cl | Br | $(CH_2)_3CH_3$ |
| F | Cl | Br | $(CH_2)_3CH_3$ |
| H | Cl | $CH_3$ | $(CH_2)_3CH_3$ |
| F | Cl | $CH_3$ | $(CH_2)_3CH_3$ |
| H | Cl | $CH_2CH_3$ | $(CH_2)_3CH_3$ |
| F | Cl | $CH_2CH_3$ | $(CH_2)_3CH_3$ |
| H | Cl | H | $CH_2CH(CH_3)_2$ |
| F | Cl | H | $CH_2CH(CH_3)_2$ |
| H | Cl | Cl | $CH_2CH(CH_3)_2$ |
| F | Cl | Cl | $CH_2CH(CH_3)_2$ |
| H | Cl | Br | $CH_2CH(CH_3)_2$ |
| F | Cl | Br | $CH_2CH(CH_3)_2$ |
| H | Cl | $CH_3$ | $CH_2CH(CH_3)_2$ |
| F | Cl | $CH_3$ | $CH_2CH(CH_3)_2$ |
| H | Cl | $CH_2CH_3$ | $CH_2CH(CH_3)_2$ |
| F | Cl | $CH_2CH_3$ | $CH_2CH(CH_3)_2$ |
| H | Cl | H | $(CH_2)_4CH_3$ |
| F | Cl | H | $(CH_2)_4CH_3$ |
| H | Cl | Cl | $(CH_2)_4CH_3$ |
| F | Cl | Cl | $(CH_2)_4CH_3$ |
| H | Cl | Br | $(CH_2)_4CH_3$ |
| F | Cl | Br | $(CH_2)_4CH_3$ |
| H | Cl | $CH_3$ | $(CH_2)_4CH_3$ |
| F | Cl | $CH_3$ | $(CH_2)_4CH_3$ |
| H | Cl | $CH_2CH_3$ | $(CH_2)_4CH_3$ |
| F | Cl | $CH_2CH_3$ | $(CH_2)_4CH_3$ |
| H | Cl | H | $(CH_2)_2CH(CH_3)_2$ |
| F | Cl | H | $(CH_2)_2CH(CH_3)_2$ |
| H | Cl | Cl | $(CH_2)_2CH(CH_3)_2$ |
| F | Cl | Cl | $(CH_2)_2CH(CH_3)_2$ |
| H | Cl | Br | $(CH_2)_2CH(CH_3)_2$ |

Tabelle B (Fortsetzung)

| $R^1$ | $R^2$ | $R^3$ | $R^4$ |
|---|---|---|---|
| F | Cl | Br | $(CH_2)_2CH(CH_3)_2$ |
| H | Cl | $CH_3$ | $(CH_2)_2CH(CH_3)_2$ |
| F | Cl | $CH_3$ | $(CH_2)_2CH(CH_3)_2$ |
| H | Cl | $CH_2CH_3$ | $(CH_2)_2CH(CH_3)_2$ |
| F | Cl | $CH_2CH_3$ | $(CH_2)_2CH(CH_3)_2$ |
| H | Cl | H | $(CH_2)_2OCH_3$ |
| F | Cl | H | $(CH_2)_2OCH_3$ |
| H | Cl | Cl | $(CH_2)_2OCH_3$ |
| F | Cl | Cl | $(CH_2)_2OCH_3$ |
| H | Cl | Br | $(CH_2)_2OCH_3$ |
| F | Cl | Br | $(CH_2)_2OCH_3$ |
| H | Cl | $CH_3$ | $(CH_2)_2OCH_3$ |
| F | Cl | $CH_3$ | $(CH_2)_2OCH_3$ |
| H | Cl | $CH_2CH_3$ | $(CH_2)_2OCH_3$ |
| F | Cl | $CH_2CH_3$ | $(CH_2)_2OCH_3$ |
| H | Cl | H | $CH(CH_3)CH_2OCH_3$ |
| F | Cl | H | $CH(CH_3)CH_2OCH_3$ |
| H | Cl | Cl | $CH(CH_3)CH_2OCH_3$ |
| F | Cl | Cl | $CH(CH_3)CH_2OCH_3$ |
| H | Cl | Br | $CH(CH_3)CH_2OCH_3$ |
| F | Cl | Br | $CH(CH_3)CH_2OCH_3$ |
| H | Cl | $CH_3$ | $CH(CH_3)CH_2OCH_3$ |
| F | Cl | $CH_3$ | $CH(CH_3)CH_2OCH_3$ |
| H | Cl | $CH_2CH_3$ | $CH(CH_3)CH_2OCH_3$ |
| F | Cl | $CH_2CH_3$ | $CH(CH_3)CH_2OCH_3$ |
| H | Cl | H | $CH_2CH=CH_2$ |
| F | Cl | H | $CH_2CH=CH_2$ |
| H | Cl | Cl | $CH_2CH=CH_2$ |
| F | Cl | Cl | $CH_2CH=CH_2$ |
| H | Cl | Br | $CH_2CH=CH_2$ |
| F | Cl | Br | $CH_2CH=CH_2$ |
| H | Cl | $CH_3$ | $CH_2CH=CH_2$ |
| F | Cl | $CH_3$ | $CH_2CH=CH_2$ |
| H | Cl | $CH_2CH_3$ | $CH_2CH=CH_2$ |
| F | Cl | $CH_2CH_3$ | $CH_2CH=CH_2$ |
| H | Cl | H | $CH_2CH=CHCH_3$ |
| F | Cl | H | $CH_2CH=CHCH_3$ |
| H | Cl | Cl | $CH_2CH=CHCH_3$ |
| F | Cl | Cl | $CH_2CH=CHCH_3$ |
| H | Cl | Br | $CH_2CH=CHCH_3$ |
| F | Cl | Br | $CH_2CH=CHCH_3$ |

Tabelle B (Fortsetzung)

| $R^1$ | $R^2$ | $R^3$ | $R^4$ |
|---|---|---|---|
| H | Cl | $CH_3$ | $CH_2CH{=}CHCH_3$ |
| F | Cl | $CH_3$ | $CH_2CH{=}CHCH_3$ |
| H | Cl | $CH_2CH_3$ | $CH_2CH{=}CHCH_3$ |
| F | Cl | $CH_2CH_3$ | $CH_2CH{=}CHCH_3$ |
| H | Cl | H | $CH_2C{\equiv}CH$ |
| F | Cl | H | $CH_2C{\equiv}CH$ |
| H | Cl | Cl | $CH_2C{\equiv}CH$ |
| F | Cl | Cl | $CH_2C{\equiv}CH$ |
| H | Cl | Br | $CH_2C{\equiv}CH$ |
| F | Cl | Br | $CH_2C{\equiv}CH$ |
| H | Cl | $CH_3$ | $CH_2C{\equiv}CH$ |
| F | Cl | $CH_3$ | $CH_2C{\equiv}CH$ |
| H | Cl | $CH_2CH_3$ | $CH_2C{\equiv}CH$ |
| F | Cl | $CH_2CH_3$ | $CH_2C{\equiv}CH$ |
| H | Cl | H | $CH_2C{\equiv}CCH_3$ |
| F | Cl | H | $CH_2C{\equiv}CCH_3$ |
| H | Cl | Cl | $CH_2C{\equiv}CCH_3$ |
| F | Cl | Cl | $CH_2C{\equiv}CCH_3$ |
| H | Cl | Br | $CH_2C{\equiv}CCH_3$ |
| F | Cl | Br | $CH_2C{\equiv}CCH_3$ |
| H | Cl | $CH_3$ | $CH_2C{\equiv}CCH_3$ |
| F | Cl | $CH_3$ | $CH_2C{\equiv}CCH_3$ |
| H | Cl | $CH_2CH_3$ | $CH_2C{\equiv}CCH_3$ |
| F | Cl | $CH_2CH_3$ | $CH_2C{\equiv}CCH_3$ |
| H | Cl | H | $CH_2Ph$ |
| F | Cl | H | $CH_2Ph$ |
| H | Cl | Cl | $CH_2Ph$ |
| F | Cl | Cl | $CH_2Ph$ |
| H | Cl | Br | $CH_2Ph$ |
| F | Cl | Br | $CH_2Ph$ |
| H | Cl | $CH_3$ | $CH_2Ph$ |
| F | Cl | $CH_3$ | $CH_2Ph$ |
| H | Cl | $CH_2CH_3$ | $CH_2Ph$ |
| F | Cl | $CH_2CH_3$ | $CH_2Ph$ |
| H | F | Cl | $CH_3$ |
| H | F | Br | $CH_3$ |
| H | F | $CH_3$ | $CH_3$ |
| H | F | Cl | $CH_2CH_3$ |
| H | F | Br | $CH_2CH_3$ |
| H | F | $CH_3$ | $CH_2CH_3$ |
| H | F | Cl | $(CH_2)_2CH_3$ |

Tabelle B (Fortsetzung)

| $R^1$ | $R^2$ | $R^3$ | $R^4$ |
|---|---|---|---|
| H | F | Br | $(CH_2)_2CH_3$ |
| H | F | $CH_3$ | $(CH_2)_2CH_3$ |
| H | F | Cl | $CH(CH_3)_2$ |
| H | F | Br | $CH(CH_3)_2$ |
| H | F | $CH_3$ | $CH(CH_3)_2$ |
| H | F | Cl | $(CH_2)_3CH_3$ |
| H | F | Br | $(CH_2)_3CH_3$ |
| H | F | $CH_3$ | $(CH_2)_3CH_3$ |
| H | F | Cl | $CH_2CH(CH_3)_2$ |
| H | F | Br | $CH_2CH(CH_3)_2$ |
| H | F | $CH_3$ | $CH_2CH(CH_3)_2$ |
| H | F | Cl | $(CH_2)_4CH_3$ |
| H | F | Br | $(CH_2)_4CH_3$ |
| H | F | $CH_3$ | $(CH_2)_4CH_3$ |
| H | F | Cl | $(CH_2)_2CH(CH_3)_2$ |
| H | F | Br | $(CH_2)_2CH(CH_3)_2$ |
| H | F | $CH_3$ | $(CH_2)_2CH(CH_3)_2$ |
| H | F | Cl | $(CH_2)_2OCH_3$ |
| H | F | Br | $(CH_2)_2OCH_3$ |
| H | F | $CH_3$ | $(CH_2)_2OCH_3$ |
| H | F | Cl | $CH(CH_3)CH_2OCH_3$ |
| H | F | Br | $CH(CH_3)CH_2OCH_3$ |
| H | F | $CH_3$ | $CH(CH_3)CH_2OCH_3$ |
| H | F | Cl | $CH_2CH{=}CH_2$ |
| H | F | Br | $CH_2CH{=}CH_2$ |
| H | F | $CH_3$ | $CH_2CH{=}CH_2$ |
| H | F | Cl | $CH_2CH{=}CHCH_3$ |
| H | F | Br | $CH_2CH{=}CHCH_3$ |
| H | F | $CH_3$ | $CH_2CH{=}CHCH_3$ |
| H | F | Cl | $CH_2C{\equiv}CH$ |
| H | F | Br | $CH_2C{\equiv}CH$ |
| H | F | $CH_3$ | $CH_2C{\equiv}CH$ |
| H | F | Cl | $CH_2C{\equiv}CCH_3$ |
| H | F | Br | $CH_2C{\equiv}CCH_3$ |
| H | F | $CH_3$ | $CH_2C{\equiv}CCH_3$ |
| H | F | Cl | $CH_2Ph$ |
| H | F | Br | $CH_2Ph$ |
| H | F | $CH_3$ | $CH_2Ph$ |

Die Zimtsäureester I bzw. die sie enthaltenden herbiziden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall

möglichst die feinste Verteilung der erfindungsgemäßen Wirkstoffe gewährleisten.

Die Verbindungen I eignen sich allgemein zur Herstellung von direkt versprühbaren Lösungen, Emulsionen, Pasten oder Öldispersionen. Als inerte Zusatzstoffe kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Chlorbenzol, Isophoron oder stark polare Lösungsmittel, wie N,N-Dimethylformamid, Dimethylsulfoxid, N-Methylpyrrolidon oder Wasser in Betracht.

Wäßrige Anwendungsformen können aus Emulsionskonzentraten, Dispersionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin-und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Die Formulierungen enthalten zwischen 0,1 und 95 Gew.%, vorzugsweise zwischen 0,5 und 90 Gew.%, Wirkstoff. Die Wirkstoffe werden dabei in einer Reinheit von 90 % bis 100 %, vorzugsweise 95 % bis 100 % (nach NMR-Spektrum) eingesetzt.

Die erfindungsgemäßen Verbindungen 1 können beispielsweise wie folgt formuliert werden:

I. Man vermischt 90 Gewichtsteile der Verbindung Nr. 1.001 mit 10 Gewichtsteilen N-Methyl-α-pyrrolidon und erhält eine Lösung, die zur Anwendung in Form kleinster Tropfen geeignet ist.

II. 20 Gewichtsteile der Verbindung Nr. 1.001 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.% des Wirkstoffs enthält.

III. 20 Gewichtsteile der Verbindung Nr. 1.001 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

IV. 20 Gewichtsteile des Wirkstoffs Nr. 1.001 werden in einer Mischung gelöst, die aus 25 Gewichtsteilen Cyclohexanon, 65 Gewichtsteilen einer Mineralölfraktion vom Siedepunkt 210 bis 280 °C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

V. 20 Gewichtsteile des Wirkstoffs Nr. 1.001 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält

man eine Spritzbrühe, die 0,1 Gew.% des Wirkstoffs enthält.

VI. 3 Gewichtsteile des Wirkstoffs Nr. 1.001 werden mit 97 Gewichtsteilen feinteiligem Kaolin vermischt. Man erhält auf diese Weise ein Stäubemittel, das 3 Gew.% des Wirkstoffs enthält.

VII. 30 Gewichtsteile des Wirkstoffs Nr. 1.001 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 20 Gewichtsteile des Wirkstoffs Nr. 1.001 werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.

Die Applikation der herbiziden Mittel bzw. der Wirkstoffe kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Bekämpfungsziel, Jahreszeit, Zielpflanzen und Wachstumsstadium 0,001 bis 3,0, vorzugsweise 0,01 bis 1,0 kg/ha aktive Substanz (a.S.).

In Anbetracht der Vielseitigkeit der Applikationsmethoden können die erfindungsgemäßen Verbindungen bzw. sie enthaltende Mittel noch in einer weiteren Zahl von Kulturplfanzen zur Beseitigung unerwünschter Pflanzen eingesetzt werden. In Betracht kommen beispielsweise folgende Kulturen:

| Botanischer Name | Deutscher Name |
|---|---|
| Allium cepa | Küchenzwiebel |
| Ananas comosus | Ananas |
| Arachis hypogaea | Erdnuß |
| Asparagus officinalis | Spargel |
| Avena sativa | Hafer |
| Beta vulgaris spp. altissima | Zuckerrübe |
| Beta vulgaris spp. rapa | Futterrübe |
| Beta vulgaris spp. esculenta | Rote Rübe |
| Brassica napus var. napus | Raps |
| Brassica napus var. napobrassica | Kohlrübe |
| Brassica napus var. rapa | Weiße Rübe |
| Brassica rapa var. silvestris | Rüben |
| Camellia sinensis | Teestrauch |
| Carthamus tinctorius | Saflor - Färberdistel |
| Carya illinoinensis | Pekannußbaum |
| Citrus limon | Zitrone |
| Citrus maxima | Pampelmuse |
| Citrus reticulata | Mandarine |
| Citrus sinensis | Apfelsine, Orange |
| Coffea arabica (Coffea canephora, Coffea liberica) | Kaffee |
| Cucumis melo | Melone |
| Cucumis sativus | Gurke |
| Cynodon dactylon | Bermudagras |
| Daucus carota | Möhre |
| Elaeis guineensis | Ölpalme |
| Fragaria vesca | Erdbeere |
| Glycine max | Sojabohne |
| Gossypium hirsutum (Gossypium arboreum, Gossypium herbaceum, Gossypium vitifolium) | Baumwolle |
| Helianthus annuus | Sonnenblume |
| Helianthus tuberosus | Topinambur |
| Hevea brasiliensis | Parakautschukbaum |

| Botanischer Name | Deutscher Name |
|---|---|
| Hordeum vulgare | Gerste |
| Humulus lupulus | Hopfen |
| Ipomoea batatas | Süßkartoffeln |
| Juglans regia | Walnußbaum |
| Lactuca sativa | Kopfsalat |
| Lens culinaris | Linse |
| Linum usitatissimum | Faserlein |
| Lycopersicon lycopersicum | Tomate |
| Malus spp. | Apfel |
| Manihot esculenta | Maniok |
| Medicago sativa | Luzerne |
| Mentha piperita | Pfefferminze |
| Musa spp. | Obst- und Mehlbanane |
| Nicotiana tabacum (N. rustica) | Tabak |
| Olea europaea | Ölbaum |
| Oryza sativa | Reis |
| Panicum miliaceum | Rispenhirse |
| Phaseolus lunatus | Mondbohne |
| Phaseolus mungo | Erdbohne |
| Phaseolus vulgaris | Buschbohnen |
| Pennisetum glaucum | Perl- oder Rohrkolbenhirse |
| Petroselinum crispum spp. tuberosum | Wurzelpetersilie |
| Picea abies | Rotfichte |
| Abies alba | Weißtanne |
| Pinus spp. | Kiefer |
| Pisum sativum | Gartenerbse |
| Prunus avium | Süßkirsche |
| Prunus domestica | Pflaume |
| Prunus dulcis | Mandelbaum |
| Prunus persica | Pfirsich |
| Pyrus communis | Birne |
| Ribes sylvestre | Rote Johannisbeere |
| Ribes uva-crispa | Stachelbeere |
| Ricinus communis | Rizinus |
| Saccharum officinarum | Zuckerrohr |
| Secale cereale | Roggen |
| Sesamum indicum | Sesam |
| Solanum tuberosum | Kartoffel |
| Sorghum bicolor (s. vulgare) | Mohrenhirse |
| Sorghum dochna | Zuckerhirse |
| Spinacia oleracea | Spinat |
| Theobroma cacao | Kakaobaum |

| Botanischer Name | Deutscher Name |
|---|---|
| Trifolium pratense | Rotklee |
| Triticum aestivum | Weizen |
| Triticum durum | Hartweizen |
| Vaccinium corymbosum | Kulturheidelbeere |
| Vaccinium vitis-idaea | Preißelbeere |
| Vicia faba | Pferdebohnen |
| Vigna sinensis (V. unguiculata) | Kuhbohne |
| Vitis vinifera | Weinrebe |
| Zea mays | Mais |

Zur Verbreiterung des Wirkungsspektrums und zur Erzielung synergistischer Effekte können die Zimtsäureester I mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate, Chinolincarbonsäurederivate, Sulfonylharnstoffderivate, Aryloxy-, Heteroaryloxyphenoxypropionsäuren sowie deren Salze, Ester und Amide und andere in Betracht.

Außerdem kann es von Nutzen sein, die neuen Verbindungen I allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs-und Spurenelementmängeln eingesetzt werden. Es können auch nichtphytotoxische Öle und Ölkonzentrate zugesetzt werden.

Synthesebeispiele

Beispiel 1

9-[E/Z-(3-{2-methoxycarbonyl-prop-1-enyl}-4-chlorphenylamino)]-8-thia-1,6-diazabicyclo[4.3.0]-nonan-7-on

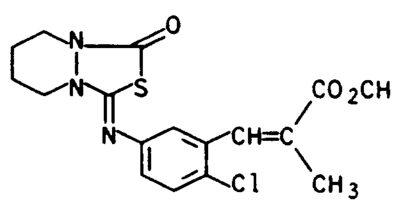

a) Eine Lösung von 468 g (1,8 mol) 2-Chlor-5-nitro-α-methylzimtsäurechlorid in 2250 ml Methanol wurde bei 5°C mit 167,4 g (1,8 mol) α-Picolin versetzt. Die Reaktionsmischung wurde 12 Stunden bei 23°C gerührt. Nach dem Abkühlen auf 0°C fiel das Produkt als Feststoff an.
Ausbeute: 294 g (64 %) 2-Chlor-5-nitro-α-methylzimtsäuremethylester; Fp. 95-96°C.
b) Eine Mischung aus 149 g Eisenpulver und 120 ml Eisessig wurde bei 70°C mit einer Mischung aus 250 ml Methanol, 350 ml Eisessig und 105 g Nitrozimtsäureester (0,4 mol) versetzt. Nach 90 Minuten bei Siedetemperatur wurde die Reaktionsmischung von Feststoffen befreit, die so erhaltene Lösung in Wasser aufgenommen und extrahiert. Aus der organischen Phase erhielt man 81,1 g (88 %) 2-Chlor-5-amino-α-methylzimtsäuremethylester (Fp. 80°C).
c) Eine Mischung aus 6,3 g (0,055 mol) Thiophosgen, 50 ml Methylenchlorid und 100 ml Wasser wurden bei 25-30°C mit 11,3 g (0,05 mol) des Produktes aus b) in 100 ml Methylenchlorid versetzt. Nach vollständiger Umsetzung (TLC) wurde die organische Phase abgetrennt. Man erhielt daraus 12,5 g (93 %) 2-Chlor-5-isothiocyanato-α-methylzimtsäuremethylester (Fp. 60-61°C).
d) Eine Lösung von 4,3 g (0,05 mol) Piperidazin in 200 ml Tetrahydrofuran wurde bei 25-30°C mit 12,0 g (0,045 mol) des Produktes aus c) in 50 ml Tetrahydrofuran versetzt. Nach vollständiger Umsetzung

(TLC) wurde das Lösungsmittel entfernt und der so erhaltene Rückstand gewaschen und getrocknet. Man erhielt 12,0 g (75 %) N-(4-Chlor-3-[2-methoxycarbonyl-prop-1-enyl]-phenylthiocarbamoyl)-hexahydropyridazin (Fp. 120-121 °C).

e) Eine Mischung von 3,5 g (0,01 mol) des Produktes aus d) und 1,7 g (0,02 mol) Pyridin in 130 ml Methylenchlorid wurde bei 25-30 °C mit 2,2 g (0,011 mol) Chlorameisensäuretrimethylester in 20 ml Methylenchlorid versetzt. Nach 2 Stunden bei 25 °C wurde mit Wasser neutral gewaschen und eingeengt. Man erhielt nach chromatographischer Reinigung 2,0 g (53 %) der Titelverbindung; (Tabelle 1, Nr. 1.001).

Die in dem Synthesebeispiel wiedergegebene Vorschrift wurde unter entsprechender Abwandlung der Ausgangsverbindungen zur Herstellung weiterer Verbindungen I benutzt.

Tabelle 1

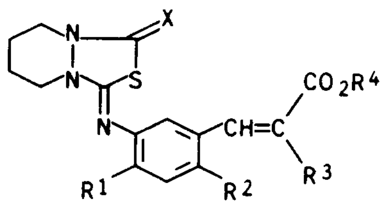

| Wirkstoff Nr. | $R^1$ | $R^2$ | $R^3$ | $R^4$ | X | physik. Daten [(Fp.(°C); IR($cm^{-1}$)] |
|---|---|---|---|---|---|---|
| 1.001 | H | Cl | $CH_3$ | $CH_3$ | O | 123-124 |
| 1.002 | H | Cl | Br | $CH_3$ | O | 1728, 1713, 1624, 1247, 1221 |
| 1.003 | H | Cl | Cl | $C_2H_5$ | O | 1717, 1619, 1248, 1221 |

Tabelle 2

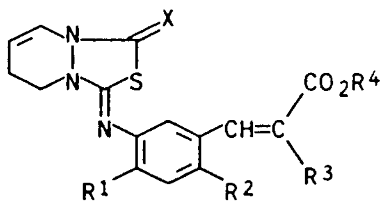

| Wirkstoff Nr. | $R^1$ | $R^2$ | $R^3$ | $R^4$ | X | physik. Daten [(Fp.(°C); IR($cm^{-1}$)] |
|---|---|---|---|---|---|---|
| 2.001 | H | Cl | Cl | $CH_3$ | O | 109-111 |
| 2.002 | H | Cl | Br | $CH_3$ | O | 105-107 |
| 2.003 | H | Cl | Br | $C(CH_3)_3$ | O | 1706, 1625, 1270, 1154 |
| 2.004 | H | Cl | Cl | $C(CH_3)_3$ | O | 1709, 1627, 1273, 1158 |
| 2.005 | H | Cl | Br | $CH(CH_3)_2$ | O | 120-121 |
| 2.006 | H | Cl | Cl | $CH(CH_3)_2$ | O | 134-135 |
| 2.007 | H | Cl | Br | $C_2H_5$ | O | 125-126 |
| 2.008 | H | Cl | Cl | $C_2H_5$ | O | 105-106 |
| 2.009 | H | Cl | Cl | $C_2H_5$ | S | 100-102 |
| 2.010 | H | Cl | Cl | $CH(CH_3)_2$ | S | 143-145 |
| 2.011 | H | Cl | Br | $CH_3$ | S | 102-103 |
| 2.012 | H | Cl | Br | $CH(CH_3)_2$ | S | 142-143 |

Anwendungsbeispiele

Die herbizide Wirkung der Zimtsäureester der Formel I ließ sich durch Gewächshausversuche zeigen:

Als Kulturgefäße dienten Plastikblumentöpfe mit lehmigem Sand mit etwa 3,0 % Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde.

Zum Zwecke der Nachauflaufbehandlung wurden die Testpflanzen je nach Wuchsform erst bei einer Wuchshöhe von 3 bis 15 cm mit den in Wasser suspendierten oder emulgierten Wirkstoffen behandelt. Die Aufwandmenge für die Nachauflaufbehandlung betrug 0,06 kg/ha a.S.

Die Pflanzen wurden artenspezifisch bei Temperaturen von 10-25°C bzw. 20-35°C gehalten. Die Versuchsperiode erstreckte sich über 2 bis 4 Wochen. Während dieser Zeit wurden die Pflanzen gepflegt und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| Abkürz. | Lateinischer Name | Deutscher Name | Englischer Name |
|---|---|---|---|
| CHEAL | Chenopodium album | Weißer Gänsefuß | lambsquarters (goosefoot) |
| GALAP | Galium aparine | Klettenlabkraut | catchweed bedstraw |
| TRZAS | Triticum aestivum | Sommerweizen | summer wheat |

Mit 0,06 kg/ha a.S. im Nachauflaufverfahren eingesetzt, lassen sich mit dem Beispiel 1.001 breitblättrige unerwünschte Pflanzen sehr gut bekämpfen bei gleichzeitiger Verträglichkeit für eine Kulturpflanze.

**Patentansprüche**
**Patentansprüche für folgende Vertragsstaaten : AT, BE, CH, DE, FR, GB, IT, LI, NL**

**1.** Zimtsäureester der allgemeinen Formel I,

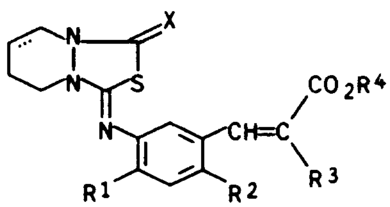

I

in der die punktierte Bindung für eine Einfach- oder Doppelbindung steht und die Substituenten folgende Bedeutung haben:

$R^1$
: Wasserstoff oder Fluor,

$R^2$
: Halogen,

$R^3$
: Wasserstoff, Halogen oder eine $C_1$-$C_4$-Alkylgruppe,

$R^4$
: Wasserstoff, eine $C_1$-$C_6$-Alkylgruppe, welche durch ein oder zwei $C_1$-$C_4$-Alkoxygruppen substituiert sein kann, eine $C_3$-$C_6$-Alkenylgruppe, eine $C_3$-$C_6$-Alkinylgruppe oder ein Benzylrest, und

X
: Sauerstoff oder Schwefel.

**2.** Verfahren zur Herstellung der Verbindungen I gemäß Anspruch 1, dadurch gekenneichnet, daß man ein Aminozimtsäurederivat der allgemeinen Formel II

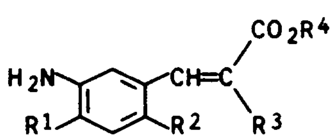

II

in an sich bekannter Weise in einem inerten organischen Lösungsmittel mit Thiophosgen in das entsprechende Isothiocyanat III

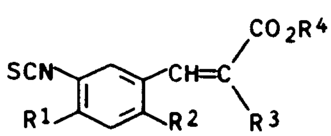

III

überführt, III anschließend an ein Tetrahydro- oder Perhydrodiazin IV

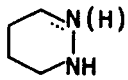

IV

addiert und den so erhaltenen Thioharnstoff V

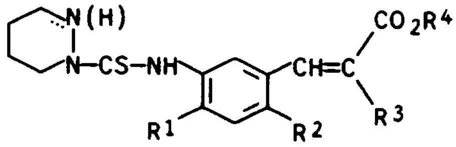

V

mit einem Phosgenierungsmittel oder Thiophosgenierungsmittel zu I cyclisiert.

**3.** Verfahren zur Herstellung der Verbindungen I gemäß Anspruch 1, dadurch gekennzeichnet, daß man ein Anilinderivat der Formel VI

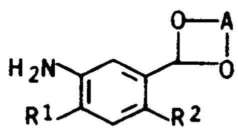

VI

in der A eine Ethylen- oder Propyleneinheit bedeutet, welche ein bis drei $C_1$-$C_3$-Alkylgruppen tragen kann, mit Thiophosgen in das entsprechende Isothiocyanat IIIa

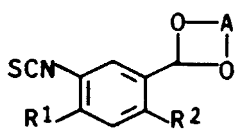

IIIa

überführt, dieses anschließend an ein Piperazin IV gemäß Anspruch 2 addiert, das so erhaltene Thioharnstoffderivat Va

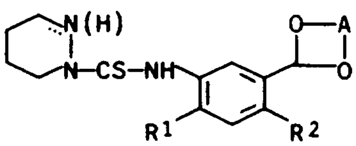

Va

unter Spaltung der Acetalgruppe in saurem Medium mit einem Phosgenierungsmittel oder Thiophosgenierungsmittel zu VII cyclisiert,

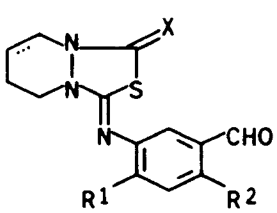

VII

und den Aldehyd VII mit einem Phosphor-Ylid der Formel VIII

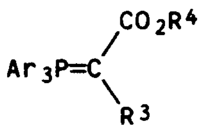

VIII

worin Ar einen Arylrest bedeutet, umsetzt.

4. Herbizide Mittel, enthaltend einen Zimtsäureester der Formel I gemäß Anspruch 1 und übliche inerte Zusatzstoffe.

5. Verfahren zur Bekämpfung unerwünschten Pflanzenwuchses, dadurch gekennzeichnet, daß man die unerwünschten Pflanzen und/oder ihren Lebensraum mit einer herbizid wirksamen Menge eines Zimtsäureesters I gemäß Anspruch 1 behandelt.

6. Verwendung der Zimtsäureester der Formel I gemäß Anspruch 1 als Herbizide.

**Patentansprüche für folgenden Vertragsstaat : ES**

1. Herbizide Mittel, enthaltend übliche inerte Zusatzstoffe und einen Zimtsäureester der Formel I

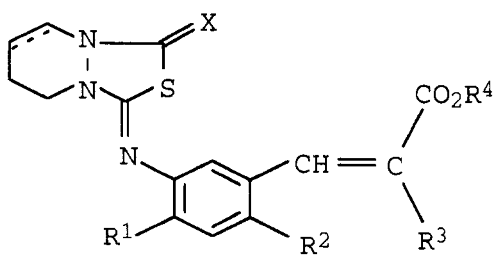

I

in der die punktierte Bindung für eine Einfach- oder Doppelbindung steht
und die Substituenten folgende Bedeutung haben:

- $R^1$ Wasser oder Fluor,
- $R^2$ Halogen,
- $R^3$ Wasserstoff, Halogen oder eine $C_1$-$C_4$-Alkylgruppe,
- $R^4$ Wasserstoff, eine $C_1$-$C_6$-Alkylgruppe, welche durch ein oder zwei $C_1$-$C_4$-Alkoxygruppen substituiert sein kann, eine $C_3$-$C_6$-Alkenylgruppe, eine $C_3$-$C_6$-Alkinylgruppe oder den Benzylrest, und
- X Sauerstoff oder Schwefel.

2. Verfahren zur Herstellung der Verbindungen I gemäß Anspruch 1, dadurch gekennzeichnet, daß man ein Aminozimtsäurederivat der Formel II

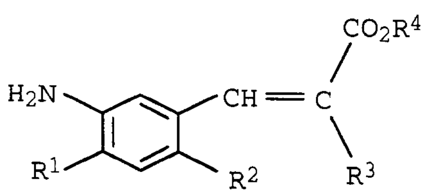

II

in an sich bekannter Weise in einem inerten organischen Lösungsmittel mit Thiophosgen in das entsprechende Isothiocyanat III

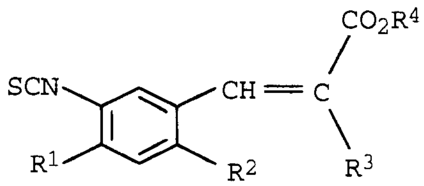

III

überführt, III anschließend an ein Tetrahydro- oder Perhydrodiazin IV

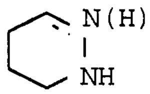

IV

addiert und den so erhaltenen Thioharnstoff V

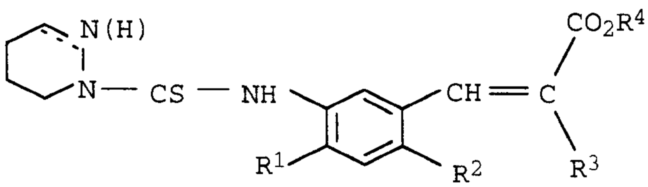

V

mit einem Phosgenierungsmittel oder Thiophosgenierungsmittel zu I cyclisiert.

3. Verfahren zur Herstellung der Verbindungen I gemäß Anspruch 1, dadurch gekennzeichnet, daß man ein Anilinderivat der Formel VI

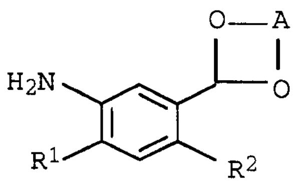

VI

in der A eine Ethylen- oder Propyleneinheit bedeutet, welche ein bis drei $C_1$-$C_3$-Alkylgruppen tragen kann, mit Thiophosgen in das entsprechende Isothiocyanat IIIa

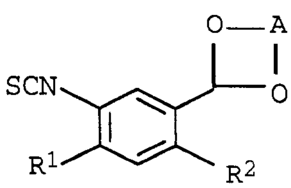

IIIa

überführt, dieses anschließend an ein Piperazin IV gemäß Anspruch 2 addiert, das so erhaltene Thioharnstoffderivat Va

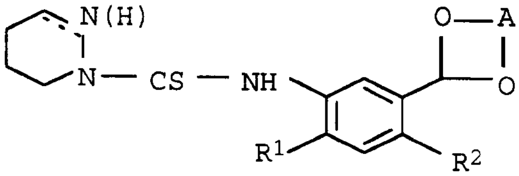

Va

unter Spaltung der Acetalgruppe in saurem Medium mit einem Phosgenierungsmittel oder Thiophosgenierungsmittel zu VII cyclisiert,

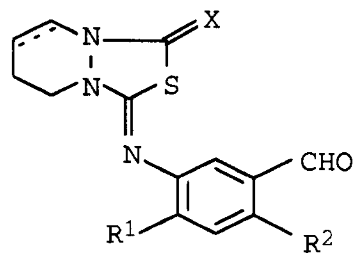

VII

und den Aldehyd VII mit einem Phosphor-Ylid der Formel VIII

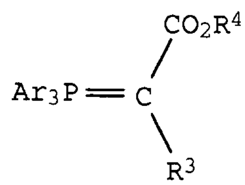

VIII

worin Ar einen Arylrest bedeutet, umsetzt.

**4.** Verfahren zur Bekämpfung von unerwünschtem Pflanzenwuchs, dadurch gekennzeichnet, daß man die unerwünschten Pflanzen und/oder ihren Lebensraum mit einer herbizid wirksamen Menge eines Zimtsäureesters I gemäß Anspruch 1 behandelt.

**5.** Verwendung der Zimtsäureester der Formel I gemäß Anspruch 1 als Herbizide.

**Claims**

**Claims for the following Contracting States : AT, BE, CH, DE, FR, GB, IT, LI, NL**

**1.** A cinnamic ester of the general formula I

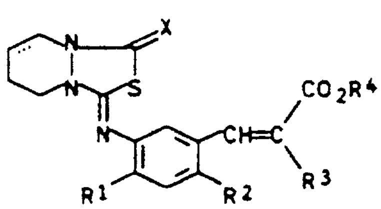

I

where the dotted bond is a single or double bond, $R^1$ is hydrogen or fluorine, $R^2$ is halogen, $R^3$ is hydrogen, halogen or $C_1$-$C_4$-alkyl, $R^4$ is hydrogen, $C_1$-$C_6$-alkyl which may be substituted by one or two $C_1$-$C_4$-alkoxy groups, or is $C_3$-$C_6$-alkenyl, $C_3$-$C_6$-alkynyl or benzyl, and X is oxygen or sulfur.

**2.** A process for the preparation of a compound I as claimed in claim 1, wherein an aminocinnamic acid derivative of the general formula II

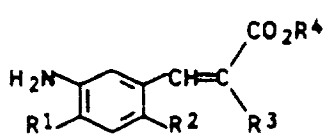

II

is converted in a conventional manner in an inert organic solvent with thiophosgene into the cor-

responding isothiocyanate III

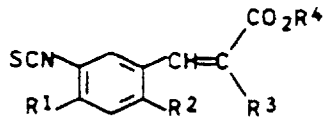

III

which is then subjected to an addition reaction with a tetrahydro- or perhydrodiazine IV

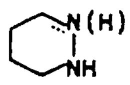

IV

and the resulting thiourea V

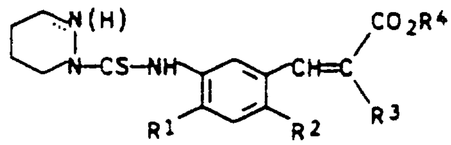

V

is subjected to a cyclization reaction with a phosgenating or thiophosgenating agent to give I.

**3.** A process for the preparation of a compound I as claimed in claim 1, wherein an aniline derivative of the formula VI

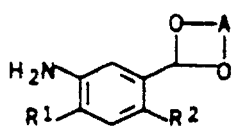

VI

where A is an ethylene or propylene unit which may carry one to three $C_1$-$C_3$-alkyl groups, is converted with thiophosgene into the corresponding isothiocyanate IIIa

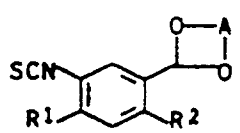

IIIa

which is then subjected to an addition reaction with a piperazine IV as claimed in claim 2, the resulting thiourea derivative Va

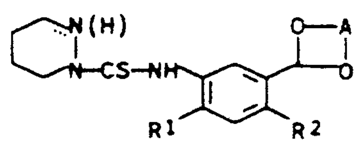

Va

is subjected to a cyclization reaction with a phosgenating or thiophosgenating agent, with cleavage of the acetal group in an acidic medium, to give VII

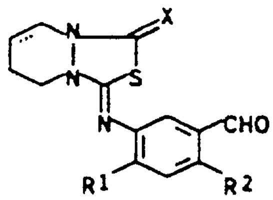

VII

and the aldehyde VII is reacted with a phosphorylide of the formula VIII

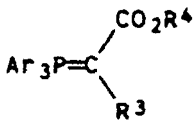

VIII

where Ar is aryl.

4. A herbicide containing a cinnamic ester of the formula I as claimed in claim 1, and conventional inert additives.

5. A method for controlling undesirable plant growth, wherein the undesirable plants and/or their habitat are treated with a herbicidally effective amount of a cinnamic ester I as claimed in claim 1.

6. The use of a cinnamic ester of the formula I as claimed in claim 1 as a herbicide.

**Claims for the following Contracting State : ES**

1. A herbicide containing conventional inert additives and a cinnamic ester of the formula I

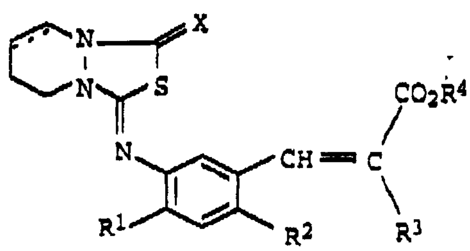

I

where the dotted bond is a single or double bond, $R^1$ is hydrogen or fluorine, $R^2$ is halogen, $R^3$ is hydrogen, halogen or $C_1$-$C_4$-alkyl, $R^4$ is hydrogen, $C_1$-$C_6$-alkyl which may be substituted by one or two $C_1$-$C_4$-alkoxy groups, or is $C_3$-$C_6$-alkenyl, $C_3$-$C_6$-alkynyl or benzyl, and X is oxygen or sulfur.

2. A process for the preparation of a compound I as claimed in claim 1, wherein an aminocinnamic acid derivative of the formula II

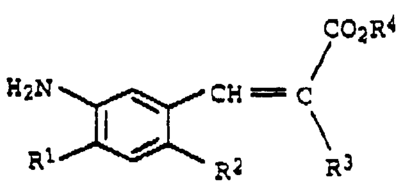

II

is converted in a conventional manner in an inert organic solvent with thiophosgene into the corresponding isothiocyanate III

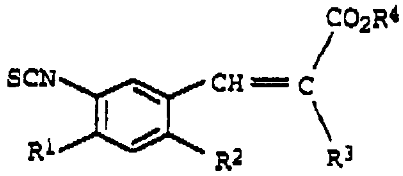

III

which is then subjected to an addition reaction with a tetrahydro- or perhydrodiazine IV

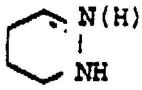

IV

and the resulting thiourea V

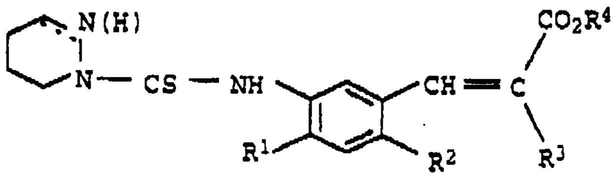

V

is subjected to a cyclization reaction with a phosgenating or thiophosgenating agent to give I.

**3.** A process for the preparation of a compound I as claimed in claim 1, wherein an aniline derivative of the formula VI

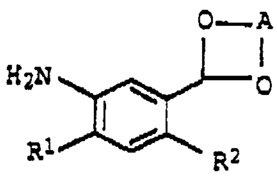

VI

where A is an ethylene or propylene unit which may carry one to three $C_1$-$C_3$-alkyl groups, is converted with thiophosgene into the corresponding isothiocyanate IIIa

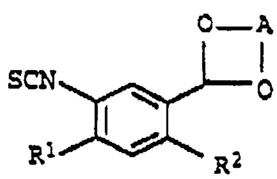

IIIa

which is then subjected to an addition reaction with a piperazine IV as claimed in claim 2, the resulting thiourea derivative Va

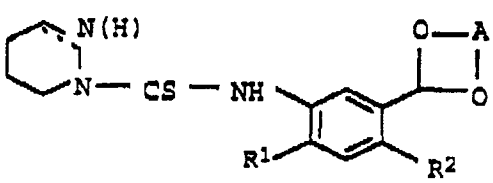

Va

is subjected to a cyclization reaction with a phosgenating or thiophosgenating agent, with cleavage of

the acetal group in an acidic medium, to give VII

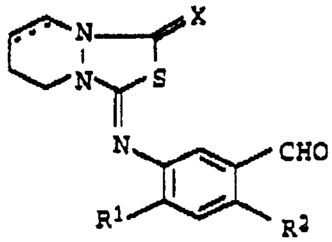

VII

and the aldehyde VII is reacted with a phosphorylide of the formula VIII

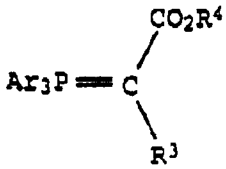

VIII

where Ar is aryl.

**4.** A method for controlling undesirable plant growth, wherein the undesirable plants and/or their habitat are treated with a herbicidally effective amount of a cinnamic ester I as claimed in claim 1.

**5.** The use of a cinnamic ester of the formula I as claimed in claim 1 as a herbicide.

**Revendications**
**Revendications pour les Etats contractants suivants : AT, BE, CH, DE, FR, GB, IT, LI, NL**

**1.** Esters cinnamiques de formule générale I

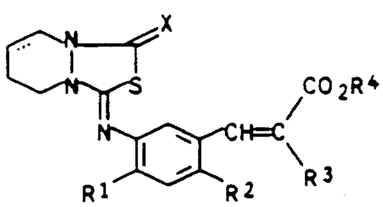

I

dans laquelle la liaison représentée en pointillé est une liaison simple ou double, et les symboles ont les significations suivantes :
$R^1$ représente l'hydrogène ou le fluor,
$R^2$ représente un halogène,
$R^3$ représente l'hydrogène, un halogène ou un groupe alkyle en C1-C4,
$R^4$ représente l'hydrogène, un groupe alkyle en C1-C6 qui peut porter un ou deux substituants alcoxy en C1-C4, un groupe alcényle en C3-C6, alcynyle en C3-C6 ou benzyle et
X représente l'oxygène ou le soufre.

**2.** Procédé de préparation des composés I de la revendication 1, caractérisé en ce que l'on convertit un dérivé d'acide aminocinnamique de formule générale II

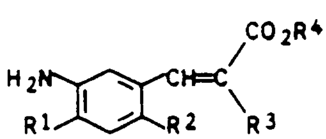

II

de manière connue en soi, dans un solvant organique inerte, à l'aide du thiophosgène, en l'isothiocyanate correspondant III

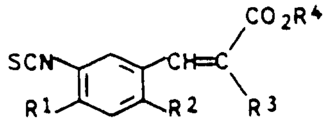

III

qu'on fixe ensuite par filtration sur une tétrahydro- ou perhydro-diazine IV

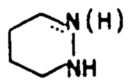

IV

ce qui donne la thiourée V

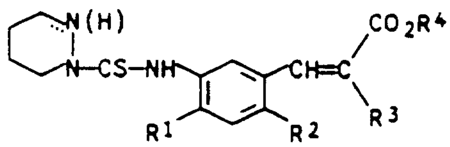

V

qu'on cyclise en I à l'aide d'un agent phosgénant ou thiophosgénant.

**3.** Procédé de préparation des composés I de la revendication 1, caractérisé en ce que l'on convertit un dérivé d'aniline de formule VI

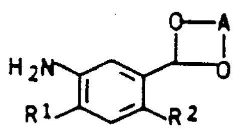

VI

dans laquelle A représente un motif éthylène ou propylène qui peut porter 1 à 3 groupes alkyle en C1-C3, à l'aide du thiophosgène, en l'isothiocyanate correspondant IIIa)

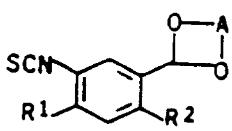

IIIa

qu'on fixe ensuite par addition sur une pipérazine IV selon la revendication 2, ce qui donne un dérivé de thiourée Va)

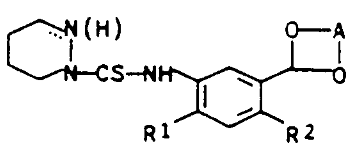

Va

qu'on cyclise avec scission du groupe acétal en milieu acide par un agent phosgénant ou thiophosgénant, ce qui donne le composé VII

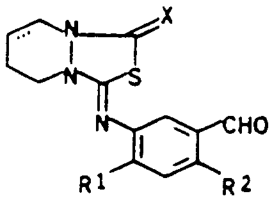

VII

aldéhyde qu'on fait réagir avec un phosphoro-ylide de formule VIII

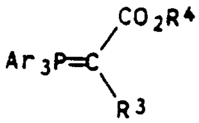

VIII

dans laquelle Ar représente un groupe aryle.

**4.** Produits herbicides contenant un ester cinnamique de formule I de la revendication 1 et des additifs inertes usuels.

**5.** Procédé pour combattre les croissances de végétaux indésirables, caractérisé en ce que l'on traite les végétaux indésirables et/ou leur habitat par une quantité herbicide efficace d'un ester cinnamique I selon la revendication 1.

**6.** Utilisation des esters cinnamiques de formule I de la revendication 1 en tant qu'herbicides.

**Revendications pour l'Etat contractant suivant : ES**

**1.** Produits herbicides contenant des additifs inertes usuels et un ester cinnamique de formule I

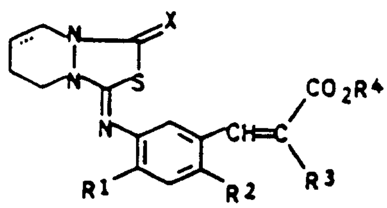

I

dans laquelle la liaison représentée en pointillé est une liaison simple ou double, et les symboles ont les significations suivantes :
$R^1$ représente l'hydrogène ou le fluor,
$R^2$ représente un halogène,
$R^3$ représente l'hydrogène, un halogène ou un groupe alkyle en C1-C4,
$R^4$ représente l'hydrogène, un groupe alkyle en C1-C6 qui peut porter un ou deux substituants alcoxy en C1-C4, un groupe alcényle en C3-C6, alcynyle en C3-C6 ou benzyle et
X représente l'oxygène ou le soufre.

**2.** Procédé de préparation des composés I de la revendication 1, caractérisé en ce que l'on convertit un dérivé d'acide aminocinnamique de formule II

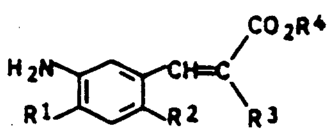

II

de manière connue en soi, dans un solvant organique inerte, à l'aide du thiophosgène, en l'isothiocya-

nate correspondant III

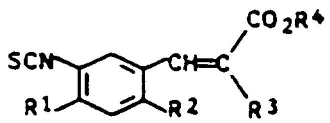

III

qu'on fixe ensuite par filtration sur une tétrahydro- ou perhydro-diazine IV

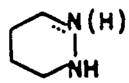

IV

ce qui donne la thiourée V

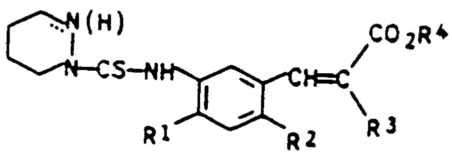

V

qu'on cyclise en I à l'aide d'un agent phosgénant ou thiophosgénant.

**3.** Procédé de préparation des composés I de la revendication 1, caractérisé en ce que l'on convertit un dérivé d'aniline de formule VI

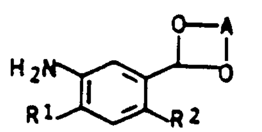

VI

dans laquelle A représente un motif éthylène ou propylène qui peut porter 1 à 3 groupes alkyle en C1-C3, à l'aide du thiophosgène, en l'isothiocyanate correspondant IIIa)

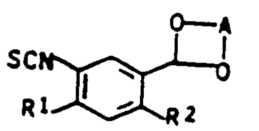

IIIa

qu'on fixe ensuite par addition sur une pipérazine IV selon la revendication 2, ce qui donne un dérivé de thiourée Va)

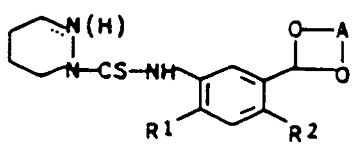

Va

qu'on cyclise avec scission du groupe acétal en milieu acide par un agent phosgénant ou thiophosgénant, ce qui donne le composé VII

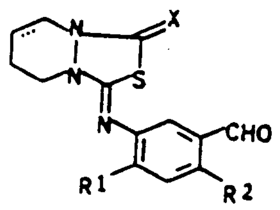

VII

aldéhyde qu'on fait réagir avec un phosphoro-ylide de formule VIII

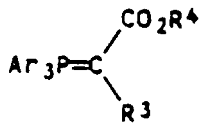

VIII

dans laquelle Ar représente un groupe aryle.

**4.** Procédé pour combattre les croissances de végétaux indésirables, caractérisé en ce que l'on traite les végétaux indésirables et/ou leur habitat par une quantité herbicide efficace d'un ester cinnamique I selon la revendication 1.

**5.** Utilisation des esters cinnamiques de formule I de la revendication 1 en tant qu'herbicides.